# NEUE EUROPÄISCHE PATENTSCHRIFT

(11) **EP 0 198 482 B2**
(45) Veröffentlichungstag und Bekanntmachung des Hinweises auf die Entscheidung über den Einspruch: **01.12.1993**
(45) Hinweis auf die Patenterteilung: 04.01.1989
(21) Anmeldenummer: 86105218.1
(22) Anmeldetag: 16.04.1986
(51) Int. Cl.: A61F 13/10

(54) **Fixier-Verband**
Fixation bandage
Bandage de fixation

(30) Priorität: 19.04.1985 DE 3514305
(43) Veröffentlichungstag der Anmeldung: 22.10.1986
(73) Patentinhaber: Habermeyer, Peter, Dr., D-70184 Stuttgart (DE)
(72) Erfinder: Habermeyer, Peter, Dr., D-70184 Stuttgart (DE)
(74) Vertreter: Richter, Werdermann & Gerbaulet

(56) Entgegenhaltungen:
- CH-A- 603 153
- GB-A- 2 019 726
- US-A- 3 906 944
- US-A- 4 469 095
- Prospekt "SCHUMACHER AKTUELL , tubigrip" der Firma Schumacher GmbH & Co. KG, mit dem Hinweis "86/1000 Druck: Papier Obermann, Krefeld"
- Prospekt "SCHUMACHER AKTUELL, tubigrip" der Firma F. & W. Schumacher KG, mit dem auf der Rückseite angebrachten Logo der Firma F. & W. Schumacher mit der Umschrift "Seit 1905"
- The Journal of Bone and Joint Surgery, Vol. 49-A, No. 4, June 1967, Seiten 750 und 751, Commander Gilchrist:" A stockinette-Velpeau for Immobilization of the Sholder Girdle"
- Rechnung der Firma Papier-Obermann vom 23.12.81

## Beschreibung

Die Erfindung betrifft einen Fixier-Verband, der insbesondere im Falle von Oberarmfrakturen und Schulterluxationen Verwendung findet.

Bei Oberarmfrakturen und auch bei Schulterluxationen ist es erforderlich, den Arm bezüglich des Oberkörpers definiert und dauerhaft zu fixieren.

Bekannte Verbände gestatten zwar eine eindeutige Fixierung des Armes des jeweiligen Patienten, haben aber nicht nur den Nachteil des vergleichsweise schwierigen und zeitaufwendigen Anlegens, sondern bringen für den Patienten auch eine Reihe von Beschwernissen mit sich, da ein Verband dieser Art etwa bis zu drei Wochen lang getragen werden muß, während dieser Zeit praktisch keine Reinigung möglich ist und insbesondere wegen unvermeidbarer Schwitzvorgänge häufig heftiger Juckreiz und stark störende Allergien auftreten.

Aus der US-A-4 469 095 ist eine elastische, schlauchförmige. Druck ausübende Arm. Bandage bekannt, deren schulterseitiges Ende mit einem schlingenförmig ausgebildeten Befestigungsband versehen ist, das mit einem Büstenhalterträgerband der jeweiligen Trägerin der Bandage verbunden werden kann, um ein Herabrutschen des Schlauchteils zu verhindern. Eine derartige Kompressions-Bandage soll die Lymphdrainage unterstützen, sie ist jedoch nicht zur Fixierung eines verletzten Armes geeignet.

Aufgabe der Erfindung ist es, einen Fixier-Verband der eingangs angegebenen Art zu schaffen, der problemfrei anlegbar, zwischenzeitlich zum Zwecke der Reinigung des Patienten einfach zu lösen und erneut zu fixieren ist, für den Patienten einen erhöhten Tragekomfort erbringt und dennoch einfach und wirtschaftlich gefertigt werden kann.

Gelöst wird diese Aufgabe bei einem Fixier-Verband mit den im Ansprüch 1 angegebe Merkmalen.

Dieser Fixier-Verband ist in einfacher Weise anlegbar, und zwar auch dann, wenn sich der Patient noch unter Narkose befindet, da dieses Anlegen im Gegensatz zu herkömmlichen Verbänden, wie z. B. dem über den gesamten Oberkörper gezogenen und dann umgeschlagenen Schlauchverband, ohne vorheriges Entfernen von Versorgungsschläuchen vorgenommen werden kann. Dieses einfache Anlegen des Fixier-Verbandes geht in der Weise vor sich, daß das Schlauchteil über Unter- und Oberarm gezogen wird, daß dann das schulterseitige Tragebend über Nacken und Brust zum abgewinkelten Unterarm geführt und dort mit einer Schlaufe um den Unterarm gelegt wird, während das handseitig vorgesehen Halteband etwa in Taillenhöhe über den Rücken geführt und mittels einer Schlaufe im unteren Bereich des Oberarms befestigt wird. Die jeweilige Schlaufenbefestigung ermöglicht ein problemfreies Spannen und auch Nachziehen des Verbandes.

Da der gesamte Oberkörper des Patienten frei von Verbandmaterial bleibt und ein kurzzeitiges Lösen des Fixierverbandes durch eine Pflegeperson ohne weiteres möglich ist, wird die Pflege des jeweiligen Patienten wesentlich erleichtert und die Gefahr des Auftretens von Ekzemen, Allergien und dergleichen praktisch beseitigt.

Eine zweckmäßige Ausgestaltung des Fixier-Verbandes zeichnet sich dadurch aus, daß der Schlauchteil aus einem radial elastischem, in Längsrichtung jedoch im wesentlichen undehnbaren Gewebematerial besteht und daß an den Schlauchteilenden jeweils ein verschließbarer Manschettenbund aus nicht dehnbarem Material vorgesehen und daran das Trageband bzw. Halteband befestigt ist. Insbesondere sind dabei die Bänder als Flachformbänder ausgebildet, die mit einer integrierten Versteifung, z. B. in Form eines Schaumstoffmaterials versehen sind. Durch die Ausgestaltung des Fixier-Verbandes wird die praktische Handhabung unmittelbar, d.h. die Art des Anlegens dieses Verbandes unmittelbar vorgegeben, wobei den einzelnen Bestandteilen, nämlich dem Schlauchteil und den Bändern die jeweiligen Funktionen eindeutig zugewiesen sind und damit diese Bestandteile auch entsprechend den jeweiligen Anforderungen konzipiert werden können.

Trägerband und Halteband sind an ihren zur Schlaufenbildung bestimmten Endbereichen mit Klettverschlußelementen versehen, wodurch das Befestigen und auch das Nachspannen des Verbandes besonders einfach und den jeweiligen Anforderungen entsprechend durchgeführt werden kann.

Eine sich durch besondere Einfachheit hinsichtlich der Herstellung auszeichnende und dennoch die Festigkeitsanforderungen besonders gut erfüllende Ausführungsform der Erfindung zeichnet sich dadurch aus, daß Trageband und Halteband zu einem einteiligen, durch das Schlauchteil geführten Band zusammengefaßt sind. Auf diese Weise werden auf den Schlauchteil praktisch keinerlei Zugkräfte ausgeübt, und es sind auch keine Verbindungsstellen zwischen Schlauchteil und Bändern erforderlich, welche größeren Zugbelastungen ausgesetzt werden müßten.

Bevorzugt ist das durchgehende Band nur im Oberarmbereich mit dem Schlauchteil fest verbunden, um eine relative Fixierung von Schlauchteil und Band sicherzustellen. Außerdem kann in diesem Falle für alle Patienten eine einheitliche Größe des Fixier-Verbandes Verwendung finden, da der handseitige Bereich auf die jeweils erforderliche Länge umgeschlagen werden kann und die Längeneinstellung des Haltebandes über die vorzugsweise vorgesehenen Klettverschlüsse im Schlaufenbereich durchführbar ist.

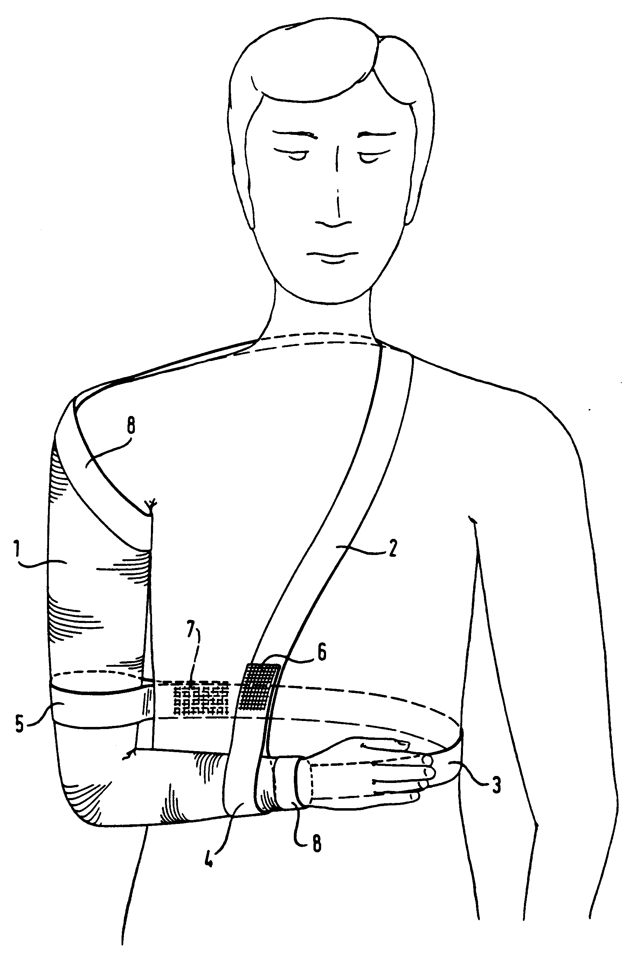

Ein Ausführungsbeispiel der Erfindung wird nachfolgend anhand der Zeichnung erläutert; in der einzigen Figur der Zeichnung ist ein FixierVerband nach der Erfindung im angelegten Zustand dargestellt.

Gemäß der Zeichnung ist über den Unter- und Oberarm des Patienten ein aus radial elastischem Gewebematerial bestehender Schlauchteil 1 gezogen. Bei dieser Ausführungsform ist am schulterseitigen Ende und am handseitigen Ende dieses Schlauchteils ein Manschettenbund 8, der vorzugsweise verstellbar ausgebildet ist, angebracht.

Am schulterseitigen Manschettenbund 8 ist ein Trageband 2 und am handseitigen Manschettenbund 8 ein Halteband 3 befestigt.

Die freien Enden dieser Bänder 2, 3 sind im dargestellten Beispiel mit vorzugsweise links und rechts verwendbaren Klettverschlüssen 6, 7 versehen, so daß einerseits mittels des Tragebandes 2 eine Schlaufe 4 um den Unterarm und mittels des Haltebandes 3 eine Schlaufe 5 um den unteren Bereich des Oberarms des Patienten gelegt werden kann.

Durch Schlaufeneinstellung über die Klettverschlüsse kann die Kraft, mittels der der Arm gegen den Oberkörper des Patienten gedrückt wird, praktisch stufenlos eingestellt werden.

Es ist ersichtlich, daß bei Verwendung eines derartigen Fixier-Verbandes der Oberkörper des Patienten trotz einwandfreier Armfixierung frei bleibt und somit keine Gefahr des Auftretens von Allergien und die Atmung behindernden Erscheinungen besteht. Ferner ist von wesentlicher Bedeutung, daß dieser Fixier-Verband auch unter Narkose komplikationsfrei angelegt werden kann und auch ein kurzzeitiges Losen und erneutes Fixieren des Armes durch das Pflegepersonal möglich ist.

Gemäß einer alternativen Ausführungsform der Erfindung, die eine besonders wirtschaftliche Herstellung des Fixier-Verbandes ermöglicht, ist ein einteiliges Halte- und Trageband vorgesehen, das durch den Schlauchteil 1 geführt ist und sämtliche Zug- bzw. Haltekräfte aufnimmt. In diesem Falle muß der Schlauchteil 1 bezüglich des Bandes lediglich zu Positionierungszwecken fixiert werden, irgendwelche kraftübertragenden Verbindungen, Nähte und dergleichen sind nicht erforderlich.

Bevorzugt wird das durchgehende Band im Oberarmbereich mittig geschlitzt, so daß eine Durchstecköffnung für den Arm besteht. Auf diese Weise läuft ein Teilband an der Vorderseite und ein Teilband an der Rückseite des Oberarmes, es ergibt sich eine symmetrische Kraftverteilung, und im Bereich des Austritts des durchgehenden Bandes aus dem Schlauchteil 1 liegt wieder ein Einzelband vor.

## Patentansprüche

1. Fixier-Verband , insbesondere für Oberarmfrakturen und Scultrluxationen , unter Verwendung eines Unterarm und Oberarm aufnehmenden , aus einem radial elastischen , in Längsrichtung jedoch jedoch im wesentlichen undehnbaren Gewebematerial bestehenden Schlauchteil (1) mit einem Trageband (2) und einem Halteband (3), und mit Verschlußorganen (6, 7) zur Bildung von Halteschlaufendadurch gekennzeichnet , daß an den Schlauchteilenden jeweils ein Manschettenbund (8) aus nicht dehnbarem Material vorgesehen ist und daran das Trageband (2) und das Halteband (3) befestig sind , wobei das Trageband (2) und das Halteband (3) als Flachformbänder mit integrierter Versteifung ausgebildet sind , und daß die Verschlußorgane (6, 7) als Klettenverschlußelemente ausgebildet sind.

2. Fixier-Verband nach Anspruch 1, dadurch gekennzeichnet, daß das Trageband (2) und das Halteband (3) zu einem einteiligen, durch das Schlauchteil (1) geführten Band zusammengefaßt sind.

3. Fixier-Verband nach Anspruch 2, dadurch gekennzeichnet, daß das durchgehende Band mit dem Schlauchteil (1) nur im Oberarmbereich fest verbunden ist.

4. Fixier-Verband nach Anspruch 2 oder 3, dadurch gekennzeichnet, daß das durchgehende Band im Oberarmbereich durch eine mittige Schlitzung mit einer Arm-Durchstecköffnung versehen ist.

## Claims

1. Fixation bandage, more particularly for fractures of the humerus and dislocations of the shoulder, in which a hose portion (1) is employed having a carrying strap (2) and a retaining strap (3) as well as being provided with closing elements (6,7) for the formation of retaining loops, said hose portion (1) accommodating the forearm and the upper arm and being radially elastic, in the longitudinal direction, however, non-extensible,
characterized in that
on the hose portion ends, in each case a cuff band (8) of non-stretchable material is provided and the carrying strap (2) and the retaining strap (3) are constructed in the form of flat straps with integrated stiffening, and in that the closing elements (6,7) are constructed in the form of Velcro strip fastener elements.

2. Fixation bandage in accordance with claim 1, characterized in that the carrier strap (2) and the holding strap (3) are combined together into a one piece strap which is led through the hose part (1).

3. Fixation bandage in accordance with claim 2, characterized in that the throughgoing strap is fixedly connected with the hose part (1) only in the upper arm region.

4. Fixation bandage in accordance with claim 2 or claim 3, characterized in that the throughgoing strap is provided in the upper arm region, by means of a central slit, with an opening through which an arm is passed.

## Revendications

1. Bandage de fixation, en particulier pour les fractures de la partie supérieure du bras et pour les luxations de l'épaule, qui utilise une partie en tuyau flexible (1), qui loge la partie inférieure et la partie supérieure du bras, en une matière tissée qui est élastique radialement tout en étant cependant essentiellement non extensible dans le sens longitudinal, avec une sangle de port (2) et une sangle de maintien (3) et avec des organes de fermeture (6, 7) pour former des des boucles de maintien,
**caractérisé en ce**
qu'il est prévu respectivement une manchette en matière non extensible respectivement aux extrémités de la partie en tuyau flexible et que la sangle de port (2) et la sangle de maintien (3) y sont fixées, la sangle de port (2) et la sangle de maintien (3) étant configurées comme des sangles de forme plate avec renforcement intégré et que les organes de fermeture (6, 7) sont configurés comme des éléments auto-agrippants.

2. Bandage de fixation selon la revendication 1, caractérisé en ce que la bande de suspension (2) et la bande de support (3) sont réunies sous forme d'une bande unique d'une pièce, passant par la partie tubulaire (1).

3. Bandage de fixation selon la revendication 2, caractérisé en ce que la bande ininterrompue est fixée à la partie tubulaire (1) uniquement au niveau de la partie supérieure du membre supérieur.

4. Bandage de fixation selon la revendication 2 ou 3, caractérisé en ce que la bande ininterrompue est, au niveau de la partie supérieure du membre supérieur, muni d'une ouverture pour le passage du bras au moyen d'une fente médiane.
